# EUROPEAN PATENT SPECIFICATION

(11) **EP 2 403 510 B1**
(45) Date of publication and mention of the grant of the patent: **26.02.2020**
(21) Application number: 09779111.5
(22) Date of filing: 05.03.2009
(51) Int. Cl.: A61K 35/745, A61K 35/747, C12N 1/20, A23L 33/135, A61P 1/00

(54) **BACTERIA STRAINS HAVING A HIGH ANTI-INFLAMMATORY ACTIVITY**
BAKTERIENSTÄMME MIT HOHER ANTIINFLAMMATORISCHER AKTIVITÄT
SOUCHES BACTÉRIENNES AYANT UNE GRANDE ACTIVITÉ ANTI-INFLAMMATOIRE

(43) Date of publication of application: 11.01.2012
(73) Proprietor: Probiotical S.p.A., 28100 Novara (IT)
(72) Inventor: MOGNA, Giovanni, I-28100 Novara (IT); STROZZI, Gian, Paolo, I-28100 Novara (IT); MOGNA, Luca, I-28100 Novara (IT)
(74) Representative: Hoffmann Eitle
(86) International application number: PCT/EP2009/052591
(87) International publication number: WO 2010/099824

(56) References cited:
- EP-A- 2 002 842
- WO-A-2006/013588
- WO-A-2007/125558
- WO-A-2008/038075
- MADSEN K L ET AL: "LACTOBACILLUS SPECIES PREVENTS COLITIS IN INTERLEUKIN 10 GENE-DEFICIENT MICE" GASTROENTEROLOGY, ELSEVIER, PHILADELPHIA, PA, vol. 116, no. 5, 1 May 1999 (1999-05-01), pages 1107-1114, XP008023587 ISSN: 0016-5085
- IMAOKA AKEMI ET AL: "Anti-inflammatory activity of probiotic Bifidobacterium: enhancement of IL-10 production in peripheral blood mononuclear cells from ulcerative colitis patients and inhibition of IL-8 secretion in HT-29 cells." WORLD JOURNAL OF GASTROENTEROLOGY : WJG 28 APR 2008, vol. 14, no. 16, 28 April 2008 (2008-04-28), pages 2511-2516, XP009117210 ISSN: 1007-9327
- PATHMAKANTHAN SHRI ET AL: "Lactobacillus plantarum 299: Beneficial in vitro immunomodulation in cells extracted from inflamed human colon." February 2004 (2004-02), JOURNAL OF GASTROENTEROLOGY AND HEPATOLOGY, VOL. 19, NR. 2, PAGE(S) 166-173 , XP009117202 ISSN: 0815-9319 the whole document
- PENA J A ET AL: "Probiotic Lactobacillus spp. Diminish Helicobacter hepaticus-Induced Inflammatory Bowel Disease in Interleukin-10-Deficient Mice" INFECTION AND IMMUNITY, AMERICAN SOCIETY FOR MICROBIOLOGY. WASHINGTON, vol. 73, no. 2, 1 February 2005 (2005-02-01), pages 912-920, XP003001378 ISSN: 0019-9567
- LEBLANC J G ET AL: "Anti-inflammatory properties of lactic acid bacteria: Current knowledge, applications and prospects" ANTI-INFECTIVE AGENTS IN MEDICINAL CHEMISTRY, BENTHAM SCIENCE PUBLISHERS, HILVERSUM, NL, vol. 7, no. 3, 1 July 2008 (2008-07-01), pages 148-154, XP009105259 ISSN: 1871-5214

## Description

The present invention relates to a composition for use in a method of treating Crohn's disease and ulcerative colitis or for use in a method of treating diarrhea and constipation. The composition comprises probiotic bacteria strains having a high anti-inflammatory activity.

It is known that probiotics are live microorganisms which when administered in adequate amounts confer a health benefits on the host. Probiotic lactobacilli and bifidobacteria are increasingly recognized as a way to prevent and/or treat intestinal disorders.

WO 2006/013588 A1 describes folic acid-producing bacterial strains belonging to the genus *Bifidobacterium,* pharmaceutical, veterinary or food formulations containing these bacteria and uses thereof. In particular, the invention relates to new bacterial strains of human origin belonging to the genus *Bifidobacterium adolescentis* species, the genus *Bifidobacterium breve* species, and the genus *Bifidobacterium pseudocatenulatum.*

Most of our encounters with antigens or infectious agents occur at mucosal surfaces, which include the surface lining the gastrointestinal, respiratory and genitourinary tracts. Since probiotics are usually absorbed orally, they are thus ideally suited to influence the immune response at the "mucosal frontier" of the gastrointestinal tract, representing more than 300 m².

The intestinal immune system forms the largest part of the immune system. It interacts with a complex antigenic load in the form of food antigens, commensal bacteria, and occasional pathogens. Dendritic cells (DC) are pivotal in earliest bacterial recognition and in shaping T cell responses. Dendritic cells sense antigen in tissues before migrating to draining lymphonodes, where they have the unique ability to activate and influence functional differentiation of naive Tcells. Signals from DC can determine whether tolerance or an active immune response occurs to a particular antigen and furthermore influence whether a Th1 or Th2 immune response predominates: DC upregulate the co-stimulatory molecules, CD80 and CD86, and produce IL-12 which contributes to a Th1 response. Further, DC may produce IL-10 and IL-4 which promote the generation of a Th2 response or regulatory T cells.

Recognition of hazardous microbes, allergens and toxins as pathogenic agents activates the gastrointestinal immune system. Antigen-specific Treg cells, which mediate oral tolerance to commensal microbes, differentiate between harmless inhabitants of the gut and pathogens. A break in the development or maintenance of oral tolerance may result in an astounding array of detrimental inflammatory disorders, including inflammatory bowel disease (IBD) and colitis.

IBD and colitis are conditions in which the immune system of patients reacts excessively to indigenous intestinal bacteria. Treg cell depletion in these disorders effectively breaches tolerance and allows for massive inflammation in the gut. In *vivo* transfer of Treg cells suppresses the development of the above diseases, through IL-10, TGF-β and CTLA-4-dependent mechanisms.

Probiotic strains can induce pro-inflammatory cytokines such as interleukin-1 (IL-1), IL-6, IL-12, tumor necrosis factor alpha (TNF-α), and gamma interferon (IFN-γ) as well as anti-inflammatory cytokines such as IL-10 and transforming growth factor β. IFN- γ and IL-12 potently augment the functions of macrophages and NK cells, which may be a possible mechanism of their anti-carcinogenic and anti-infectious activity. On the other hand, induction of IL-10 and transforming growth factor β is assumed to participate in the down-regulation of inflammation, since these cytokines can inhibit the functions of macrophages and T cells and promote the development of regulatory T cells. IL-10 is produced by many cells, including Th2 cells, DCs, monocytes, B cells, keratinocytes and regulatory T cells; it has an anti-inflammatory effect and primarily acts to inhibit the Th1 response. IL-10 drives the generation of a CD4+ T-cell subset, designated T regulatory cells 1 (Tr1), suppressing antigen-specific immune responses and actively down-regulates a pathological immune response *in vivo.*

Several intestinal conditions are under the umbrella of "Inflammatory Bowel Disease (IBD)", including Crohn's disease, ulcerative colitis and pouchitis.

In inflammatory bowel disease, IL-10 is a cytokine of particular therapeutic interest since it has been shown in animal models that interleukin (IL)-10(-/-) mice spontaneously develop intestinal inflammation.

It has been shown in animal models that probiotic strains displaying an *in vitro* potential to induce higher levels of the anti-inflammatory cytokine IL-10 and lower levels of the inflammatory cytokine IL-12, offer the best protection against *in vivo* colitis in the model.

EP 2 002 842 A2 describes a method for producing an interleukin production regulator having both an effect of maintaining or promoting the production of interleukin-10 and an effect of maintaining or inhibiting the production of interleukin-12. The method comprises the step of disrupting a cell of a microorganism belonging to the genus *Bifidobacterium;* an interleukin production regulator produced by the method; and a pharmaceutical and a food, each comprising the interleukin production regulator.

Probiotic-mediated immunomodulation represents an interesting option in the management of IBD and it was shown that both the systemic and mucosal immune systems can be modulated by orally delivered bacteria. However, not all candidate probiotics have been proven equally efficient due to the differences in survival and persistence of the strain in the gastro-intestinal tract, and/or to strain-specific interactions of the probiotic with the host immune system. The selection of a successful protective strain may therefore rely on the proper screening of a large number of candidate strains for their technological and immunomodulatory performance.

Therefore, it remains the need to isolate and select bacteria strains having a marked anti-inflammatory activity. In particular, it remains the need to isolate and select specific bacteria strains as strongly inducers of IL-10 production. Further, it remains the need to isolate and select bacteria strains with a low capability to stimulate the production of the pro-inflammatory Il-12, thus leading to a IL-10/IL-12 ratio at least bigger than one. Finally it remains the need to find out and select bacteria strains which show high persistence in the gastro-intestinal tract due to their resistance to gastric juice, bile salts, pancreatic secretion and to adhesion to gut wall. Last but not least it is important to select bacteria strains without acquired antibiotic resistances.

The Applicant has selected a group of bacteria strains which are able to solve the outstanding problems present in the prior art. As a result, the present invention relates to a composition for use in a method of treating Crohn's disease and ulcerative colitis or for use in a method of treating diarrhea and constipation,
wherein the composition is administered orally,
wherein the composition comprises live bacteria, and
wherein the live bacteria belong to a strain selected from the group consisting of *Lactobacillus paracasei* LMG P-21380, *Lactobacillus plantarum* LMG P-21021, *Bifidobacterium lactis* LMG P-21384, and *Bifidobacterium breve* DSM 16604, as inducer of Interleukin-10.

The Applicant has tested bacteria strains belonging to the following species: *L. acidophilus, L. crispatus, L. gasseri, L. delbrueckii, L. salivarius, L. casei, L. paracasei, L. plantarum, L. rhamnosus, L. reuteri, L. brevis, L. buchneri, L. fermentum, B. adolescentis, B. angulatum, B. bifidum, B. breve, B. catenulatum, B. infantis, B. lactis, B. longum, B. pseudocatenulatum,* and *S. thermophilus.*

Table 1 shows a group of bacteria strains which find a valid application in the contest of the present invention.

**TABLE 1**

| **N°** | **Bacterium strain** | **Deposit number** | **Deposit date** | **Depositor** |
|---|---|---|---|---|
| 1 | *Streptococcus thermophilus B39* | LMG P-18383 | 5.05.1998 | ANIDRAL S.R.L. |
| 2 | *Streptococcus thermophilus T003* | LMG P-18384 | 5.05.1998 | ANIDRAL S.R.L. |
| 3 | *Lactobacillus pentosus 9*/*1 ei* | LMG P-21019 | 16.10.2001 | MOFIN S.R.L. |
| 4 | *Lactobacillus plantarum 776*/*1 bi* | LMG P-21020 | 16.10.2001 | MOFIN S.R.L. |
| 5 | *Lactobacillus plantarum 476LL 20 bi* | LMG P-21021 | 16.10.2001 | MOFIN S.R.L. |
| 6 | *Lactobacillus plantarum PR ci* | LMG P-21022 | 16.10.2001 | MOFIN S.R.L. |
| 7 | *Lactobacillus plantarum 776*/*2 hi* | LMG P-21023 | 16.10.2001 | MOFIN S.R.L. |
| 8 | *Lactobacillus casei ssp. paracasei 181A*/*3 aiai* | LMG P-21380 | 31.01.2002 | ANIDRAL S.R.L. |
| 9 | *Lactobacillus belonging to the acidophilus group 192A*/*1 aiai* | LMG P-21381 | 31.01.2002 | ANIDRAL S.R.L. |
| 10 | *Bifidobacterium longum 175A*/*1 aiai* | LMG P-21382 | 31.01.2002 | ANIDRAL S.R.L. |
| 11 | *Bifidobacterium breve 195A*/*1 aici* | LMG P-21383 | 31.01.2002 | ANIDRAL S.R.L. |
| 12 | *Bifidobacterium lactis 32A*/*3 aiai* | LMG P-21384 | 31.01.2002 | ANIDRAL S.R.L. |
| 13 | *Lactobacillus plantarum 501*/*2 gi* | LMG P-21385 | 31.01.2002 | MOFIN S.R.L. |
| 14 | *Lactococcus lactis ssp. lactis 501*/*4 hi* | LMG P-21387 | 15.03.2002 | MOFIN S.R.L. |
| 15 | *Lactococcus lactis ssp. lactis 501*/*4 ci* | LMG P-21388 | 31.01.2002 | MOFIN S.R.L. |
| 16 | *Lactobacillus plantarum 501*/*4 li* | LMG P-21389 | 15.03.2002 | MOFIN S.R.L. |
| 17 | *Streptococcus thermophilus GB1* | DSM 16506 | 18.06.2004 | PROBIOTICAL S.p.A. |
| 18 | *Streptococcus thermophilus GB5* | DSM 16507 | 18.06.2004 | PROBIOTICAL S.p.A. |
| 19 | *Bifidobacterium longum BL 03* | DSM 16603 | 20.07.2004 | PROBIOTICAL S.p.A. |
| 20 | *Bifidobacterium breve BR 03* | DSM 16604 | 20.07.2004 | PROBIOTICAL S.p.A. |
| 21 | *Lactobacillus casei ssp. rhamnosus LR 04* | DSM 16605 | 20.07.2004 | PROBIOTICAL S.p.A. |
| 22 | *Lactobacillus delbrueckii ssp. bulgaricus LDB 01* | DSM 16606 | 20.07.2004 | PROBIOTICAL S.p.A. |
| 23 | *Lactobacillus delbrueckii ssp. bulgaricus LDB 02* | DSM 16607 | 20.07.2004 | PROBIOTICAL S.p.A. |
| 24 | *Streptococcus thermophilus Y02* | DSM 16590 | 20.07.2004 | PROBIOTICAL S.p.A. |
| 25 | *Streptococcus thermophilus Y03* | DSM 16591 | 20.07.2004 | PROBIOTICAL S.p.A. |
| 26 | *Streptococcus thermophilus Y04* | DSM 16592 | 20.07.2004 | PROBIOTICAL S.p.A. |
| 27 | *Streptococcus thermophilus Y05* | DSM 16593 | 20.07.2004 | PROBIOTICAL S.p.A. |
| 28 | *Bifidobacterium adolescentis BA 03* | DSM 16594 | 21.07.2004 | PROBIOTICAL S.p.A. |
| 29 | *Bifidobacterium adolescentis BA 04* | DSM 16595 | 21.07.2004 | PROBIOTICAL S.p.A. |
| 30 | *Bifidobacterium breve BR 04* | DSM 16596 | 21.07.2004 | PROBIOTICAL S.p.A. |
| 31 | *Bifidobacterium pseudocatenulatum BP 01* | DSM 16597 | 21.07.2004 | PROBIOTICAL S.p.A. |
| 32 | *Bifidobacterium pseudocatenulatum BP 02* | DSM 16598 | 21.07.2004 | PROBIOTICAL S.p.A. |
| 33 | *Staphylococcus xylosus SX 01* | DSM 17102 | 01.02.2005 | PROBIOTICAL S.p.A. |
| 34 | *Bifidobacterium adolescentis BA 02* | DSM 17103 | 01.02.2005 | PROBIOTICAL S.p.A. |
| 35 | *Lactobacillus plantarum LP 07* | DSM 17104 | 01.02.2005 | PROBIOTICAL S.p.A. |
| 36 | *Streptococcus thermophilus YO8* | DSM 17843 | 21.12.2005 | PROBIOTICAL S.p.A. |
| 37 | *Streptococcus thermophilus YO9* | DSM 17844 | 21.12.2005 | PROBIOTICAL S.p.A. |
| 38 | *Streptococcus thermophilus YO100* | DSM 17845 | 21.12.2005 | PROBIOTICAL S.p.A. |
| 39 | *Lactobacillus fermentum LF06* | DSM 18295 | 24.05.2006 | PROBIOTICAL S.p.A. |
| 40 | *Lactobacillus fermentum LF07* | DSM 18296 | 24.05.2006 | PROBIOTICAL S.p.A. |
| 41 | *Lactobacillus fermentum LF08* | DSM 18297 | 24.05.2006 | PROBIOTICAL S.p.A. |
| 42 | *Lactobacillus fermentum LF09* | DSM 18298 | 24.05.2006 | PROBIOTICAL S.p.A. |
| 43 | *Lactobacillus gasseri LGS01* | DSM 18299 | 24.05.2006 | PROBIOTICAL S.p.A. |
| 44 | *Lactobacillus gasseri LGS02* | DSM 18300 | 24.05.2006 | PROBIOTICAL S.p.A. |
| 45 | *Lactobacillus gasseri LGS03* | DSM 18301 | 24.05.2006 | PROBIOTICAL S.p.A. |
| 46 | *Lactobacillus gasseri LGS04* | DSM 18302 | 24.05.2006 | PROBIOTICAL S.p.A. |
| 47 | *Bifidobacterium adolescentis EI-3* | DSM 18350 | 15.06.2006 | PROBIOTICAL S.p.A. |
| 48 | *Bifidobacterium adolescentis EI-15* | DSM 18351 | 15.06.2006 | PROBIOTICAL S.p.A. |
| 49 | *Bifidobacterium adolescentis EI-18* | DSM 18352 | 15.06.2006 | PROBIOTICAL S.p.A. |
| 50 | *Bifidobacterium catenulatum EI-20* | DSM 18353 | 15.06.2006 | PROBIOTICAL S.p.A. |
| 51 | *Streptococcus thermophilus FRai* | DSM 18613 | 13.09.2006 | MOFIN S.R.L. |
| 52 | *Streptococcus thermophilus LB2bi* | DSM 18614 | 13.09.2006 | MOFIN S.R.L. |
| 53 | *Streptococcus thermophilus LRci* | DSM 18615 | 13.09.2006 | MOFIN S.R.L. |
| 54 | *Streptococcus thermophilus FP4* | DSM 18616 | 13.09.2006 | MOFIN S.R.L. |
| 55 | *Streptococcus thermophilus ZZ5F8* | DSM 18617 | 13.09.2006 | MOFIN S.R.L. |
| 56 | *Streptococcus thermophilus TEO4* | DSM 18618 | 13.09.2006 | MOFIN S.R.L. |
| 57 | *Streptococcus thermophilus Slci* | DSM 18619 | 13.09.2006 | MOFIN S.R.L. |
| 58 | *Streptococcus thermophilus 641bi* | DSM 18620 | 13.09.2006 | MOFIN S.R.L. |
| 59 | *Streptococcus thermophilus 277A*/*1ai* | DSM 18621 | 13.09.2006 | MOFIN S.R.L. |
| 60 | *Streptococcus thermophilus 277A*/*2ai* | DSM 18622 | 13.09.2006 | MOFIN S.R.L. |
| 61 | *Streptococcus thermophilus IDC11* | DSM 18623 | 13.09.2006 | MOFIN S.R.L. |
| 62 | *Streptococcus thermophilus ML3di* | DSM 18624 | 13.09.2006 | MOFIN S.R.L. |
| 63 | *Streptococcus thermophilus TEO3* | DSM 18625 | 13.09.2006 | MOFIN S.R.L. |
| 64 | *Streptococcus thermophilus G62* | DSM 19057 | 21.02.2007 | MOFIN S.R.L. |
| 65 | *Streptococcus thermophilus G1192* | DSM 19058 | 21.02.2007 | MOFIN S.R.L. |
| 66 | *Streptococcus thermophilus GB18* | DSM 19059 | 21.02.2007 | MOFIN S.R.L. |
| 67 | *Streptococcus thermophilus CCR21* | DSM 19060 | 21.02.2007 | MOFIN S.R.L. |
| 68 | *Streptococcus thermophilus G92* | DSM 19061 | 21.02.2007 | MOFIN S.R.L. |
| 69 | *Streptococcus thermophilus G69* | DSM 19062 | 21.02.2007 | MOFIN S.R.L. |
| 70 | *Streptococcus thermophilus YO 10* | DSM 19063 | 21.02.2007 | PROBIOTICAL S.p.A. |
| 71 | *Streptococcus thermophilus YO 11* | DSM 19064 | 21.02.2007 | PROBIOTICAL S.p.A. |
| 72 | *Streptococcus thermophilus YO 12* | DSM 19065 | 21.02.2007 | PROBIOTICAL S.p.A. |
| 73 | *Streptococcus thermophilus YO 13* | DSM 19066 | 21.02.2007 | PROBIOTICAL S.p.A. |
| 74 | *Weissella ssp. WSP 01* | DSM 19067 | 21.02.2007 | PROBIOTICAL S.p.A. |
| 75 | *Weissella ssp. WSP 02* | DSM 19068 | 21.02.2007 | PROBIOTICAL S.p.A. |
| 76 | *Weissella ssp. WSP 03* | DSM 19069 | 21.02.2007 | PROBIOTICAL S.p.A. |
| 77 | *Lactobacillus plantarum LP 09* | DSM 19070 | 21.02.2007 | PROBIOTICAL S.p.A. |
| 78 | *Lactococcus lactis NS 01* | DSM 19072 | 21.02.2007 | PROBIOTICAL S.p.A. |
| 79 | *Lactobacillus plantarum LP 10* | DSM 19071 | 21.02.2007 | PROBIOTICAL S.p.A. |
| 80 | *Lactobacillus fermentum LF 10* | DSM 19187 | 20.03.2007 | PROBIOTICAL S.p.A. |
| 81 | *Lactobacillus fermentum LF 11* | DSM 19188 | 20.03.2007 | PROBIOTICAL S.p.A. |
| 82 | *Lactobacillus casei ssp. rhamnosus LR 05* | DSM 19739 | 27.09.2007 | PROBIOTICAL S.p.A. |
| 83 | *Bifidobacterium bifidum BB01* | DSM 19818 | 30.10.2007 | PROBIOTICAL S.p.A. |
| 84 | *Lactobacillus delbrueckii LD 01* | DSM 19948 | 28.11.2007 | PROBIOTICAL S.p.A. |
| 85 | *Lactobacillus delbrueckii LD 02* | DSM 19949 | 28.11.2007 | PROBIOTICAL S.p.A. |
| 86 | *Lactobacillus delbrueckii LD 03* | DSM 19950 | 28.11.2007 | PROBIOTICAL S.p.A. |
| 87 | *Lactobacillus delbrueckii LD 04* | DSM 19951 | 28.11.2007 | PROBIOTICAL S.p.A. |
| 88 | *Lactobacillus delbrueckii LD 05* | DSM 19952 | 28.11.2007 | PROBIOTICAL S.p.A. |
| 89 | *Lactobacillus acidophilus LA 02* | DSM 21717 | 06.08.2008 | PROBIOTICAL S.P.A. |
| 90 | *Lactobacillus paracasei LPC 08* | DSM 21718 | 06.08.2008 | PROBIOTICAL S.P.A. |
| 91 | *Lactobacillus pentosus LPS 01* | DSM 21980 | 14.11.2008 | PROBIOTICAL S.P.A. |
| 92 | *Lactobacillus rhamnosus LR 06* | DSM 21981 | 14.11.2008 | PROBIOTICAL S.P.A. |

Of the bacteria mentioned above, the present invention makes particular use of *Lactobacillus paracasei* LMG P-21380, *Lactobacillus plantarum* LMG P-21021, *Bifidobacterium lactis* LMG P-21384, and *Bifidobacterium breve* DSM 16604.

The bacteria strains according to the present invention contribute to the prevention or treatment of immune diseases including autoimmune diseases such as inflammatory bowel diseases, and contribute to maintenance of the immunological homeostasis (health maintenance) of mammals such as human beings, domestic animals, and pet animals.

In other words, the bacteria strains are high in safety and can be orally administered. Thus, the microorganisms are useful in that immunoregulatory cells can efficiently induced in the body by making use of the microorganism as an active ingredient of pharmaceutical products, a food product, and the animal feeding stuff.

Other aspects and features of the invention will be more fully apparent from the following disclosure and appended claims.
Figure 1 is a diagram showing an amount (pg/ml) of cytokine IL-10 production. Strain-specific patterns of IL-10 and IL-12 release for different microorganism strains.
Figure 2 is a diagram showing the IL-10/IL-12 ratio. Strain-specific IL-10/IL-12 ratio for different microorganism strains.

The invention will be fully described by means of the following description without any limiting effects.

In the present invention a bacterium strain is selected from the group consisting of *L. paracasei* LMG P-21380, *L. plantarum* LMG P-21021, *Bifidobacterium lactis* LMG P-21384, *Bifidobacterium breve* DSM 16604, which induces the production of Interleukin-10. Further, said bacteria strains exhibit a IL-10/IL-12 ratio comprised from bigger than 1 and less than 150, preferably comprised from 10 and 100, more preferably comprised from 30 and 60.

Advantageously, the bacteria strain is *Bifidobacterium breve* DSM 16604 which induces the production of Interleukin-10 and exhibits a IL-10/Il-12 ratio which is comprised from 50 and 100, preferably from 70 and 80.

The bacteria strains are in the form of live bacteria.

In a preferred embodiment the composition is in the form of a food product.

The composition of the invention is for use as a medicament for the prevention or treatment of inflammatory conditions of the large intestine and small intestine or for the prevention or treatment of functional bowel disorders. The inflammatory conditions are selected from Crohn's disease and ulcerative colitis while the functional bowel disorders are selected from diarrhea and constipation.

Said bacteria strains induce the production of Interleukin-10. Further, said bacteria strains exhibit a IL-10/IL-12 ratio comprised from bigger than 1 and less than 150, preferably comprised from 10 and 100, more preferably comprised from 30 and 60. Advantageously, the bacteria strain is *Bifidobacterium breve* DSM 16604 which induces the production of Interleukin-10 and exhibits an IL-10/Il-12 ratio which is comprised from 50 and 100, preferably from 70 and 80.

In a preferred embodiment, the composition contains bacteria strains , as an active ingredients, in an amount comprised from 1x10⁶ to 1x10¹¹ CFU/g, respect to the weight of the composition, preferably from 1x10⁸ to 1x10¹¹ CFU/g.

In a preferred embodiment, the composition contains bacteria strains , as an active ingredient, in an amount comprised 1x10⁶ to 1x10¹¹ CFU/dose, preferably from 1x10⁸ to 1x10¹⁰ CFU/dose.

The dose may be of 1 g, 3 g, 5 g, and 10 g.

The composition may further comprise additives and co-formulates pharmaceutically acceptable.

The composition of the present invention may include vitamins (for example folic acid, riboflavin, vitamine E, ascorbic acid), antioxidants compounds (for example polphenols, flavonoids and proanthocyanidines), aminoacid (for example glutamin, metionin) and also mineral (for example selenium and zinc).

In another particularly preferred embodiment, the composition of the present invention further includes at least a substance having prebiotic properties in an amount comprised from 1 to 30% by weight, respect to the total weight composition, preferably from 5 to 20% by weight.

Said prebiotic substance preferably includes carbohydrates which are not digested and absorbed by the organism. Said carbohydrates are preferably selected from: fructo-oligosaccharides (or FOS), short-chain fructo-oligosaccharides, inulin, isomalt-oligosaccharides, pectins, xylo-oligosaccharides (or XOS), chitosan-o- ligosaccharides (or COS), beta-glucans, arabic gum modified and re-sistant starches, polydextrose, D-tagatose, acacia fibers, bambu', carob, oats, and citrus fibers. Particularly preferred prebiotics are the short-chain fructo-oligosaccharides (for simplicity shown herein- below as FOSs-c.c); said FOSs-c.c. are not digestable glucides, generally obtained by the conversion of the beet sugar and including a saccharose molecule to which three glucose molecules are bonded.

In a preferred embodiment the bacteria strain *Bifidobacterium breve* DSM 16604 is in combination with at least one bacteria strains selected from the group consisting of *L. paracasei* LMG P-21380, *L. plantarum* LMG P-21021, and *Bifidobacterium lactis* LMG P-21384.

Although a number of bacteria were tested and are described below, the invention is subject to the appended claims and makes use of *Lactobacillus paracasei* LMG P-21380, *Lactobacillus plantarum* LMG P-21021, *Bifidobacterium lactis* LMG P-21384, and *Bifidobacterium breve* DSM 16604.

The following bacteria strains have been tested. Three *Lactobacillus* strains: *L. rhamnosus* (LR04) DSM 16605, *L. paracasei* (LPC 00) LMG P-21380, *L. plantarum* (LP 01) LMG P-21021, and two *Bifidobacterium* strains: *B. lactis* (BS 01) LMG P-21384, and *B. breve* (BR 03) DSM 16604 belonging to the most representative species of probiotic bacteria, were selected based on their resistance to acid, digestive enzyme, and bile and other characteristics such as antibiotic resistance and safety of use.

Living (viable) and dead (killed) bacteria samples were prepared starting from frozen stocks collection as follows. Pure *Lactobacillus* strains were cultured in de Man, Rogosa and Sharpe broth (MRS, DeMan et al. 1960) while *Bifidobacterium* strains, were cultured in MRS or Tryptone Phytone Yeast broth (TPY, Scardovi 1986), supplemented with 0.05% L-cysteine-hydrochloride. The cultures were prepared at 37°C under anaerobic conditions for 16-22 hours. All bacteria were harvested by centrifugation (3000g for 15 min) during exponential and/ or stationary growth phase in order to collect cells. Pelleted bacteria were then washed in phosphate buffered saline (PBS) and concentration was determined by means of colony-forming unit (CFU) counting.

With reference to the preparation of living (viable) bacteria samples, washed pelleted bacteria were diluted to a final working concentration of 1x10⁹CFU/mL in PBS containing 20% glycerol and stored at -80°C until used for assay. Alternatively bacteria could be diluted in RPMI-1640 and the suspension aliquoted and stored at -20°C.

Survival of bacteria upon freezing and thawing was determined by amount of live bacteria by means of colony-forming unit (CFU) counting and/ or with staining for cFDA (live) and PI (dead). For all strains tested, >80% was alive upon thawing. The percentage of viability was not dependent on the time of storage. One fresh aliquot was thawed for every new experiment to avoid variability in the cultures between experiments.

With reference to the preparation of the dead bacteria samples, one of the following procedures may be used. Heatkilled bacterial cultures were prepared by heating the above washed pelleted bacteria resuspended in distilled water at 100°C for 30 min. Alternatively bacteria can be γ-irradiated or sonicated. Apart from one of the above procedures used for having a dead bacteria sample, the above sample may be treated in a liquid form or in a freeze-dried one.

The bacteria strains of the present invention were co-cultured with PBMCs (Peripheral Blood Mononuclear Cells) in order to study the specific capability to induce cytokine production by immunopotent cells.

PBMCs were isolated from peripheral blood of healthy donor as described. Briefly, after Ficoll gradient centrifugation, mononuclear cells were collected, washed in PBS and adjusted to 2x10⁶ cells/mL in a complete medium consisting of RPMI 1640 supplemented with L-glutamin (300 mg/l), penicillium (100 U/ml), streptomycin (64 U/ml and 10% heat inactivated FCS (Fetal Calf Serum).

Alternatively a RPMI complete medium can also be obtained by RPMI-1640 supplemented with L-glutamin (300 mg/l), gentamicin (500 µg/mL), penicillin (100 U/mL), streptomycin (64 U/ml) and 20% heat-inactivated human AB serum or 10% FCS.

Monocytes can be purified from PBMCs by negative magnetic cell sorting. The positively selected cells can be used as source of peripheral blood lymphocytes (PBLs). Monocytes as well as PBLs can be counted and resuspended at a concentration of 5x10⁶ cells/mL in complete RPMI medium. For mononuclear cells (PBMCs, Monocytes and PBLs) cryopreservation in liquid nitrogen, that cells, collected after Ficoll gradient centrifugation, were resuspended at a concentration of 1x106 cells/mL in a complete medium consisting of RPMI 1640 supplemented with 10% DMSO (Dimethyl sulfoxide).

PBMCs cultures were set up in duplicate or triplicate in 96-well flat or round-bottom polystyrene microtitre plates. All cultures contained 0.1- 0.5x10⁶ PBMCs (or monocytes or PBLs) in complete medium. PBMCs were cultured in medium only or stimulated with phytoemoglutinine (PHA) at a final concentration of 50µg/mL or lipopolisaccharides (LPS) at a final concentration of 0,5- 1µg/mL. The co-cultures with the live bacteria samples were obtained by adding a thawed aliquot of live bacteria sample to the PBMCs cultures having a cell: bacteria ratio of 1:1, 1:10 or 1:200.

The above bacteria-cell optimal concentration can be determined after proliferation test with different relative concentration (for example varying concentrations of bacterial cell fractions from 10⁶ to 10⁹ CFU/ml).

With reference to the co-cultures test with dead bacteria samples, PBMCs were cultured with 5-20 µg/mL (preferably 10 µg/mL) of dead bacteria samples (heatkilled, γ-irradiated or sonicated) in freezed-dried form or with dead bacteria samples in the liquid form having a bacteria: cell ratio from 50:1 to 250:1 (preferably 200:1).

Control cultures contained unstimulated PBMCs, PHA-stimulated PBMCs, monocytes, PBLs all without bacteria strains or live bacteria sample only.

The plates were incubated at 37°C in 5% CO₂. The supernatants of cultures were collected at 24, 48, 72 hours and 5 days, clarified by centrifugation and stored at -20°C until cytokine analysis. Neither medium acidification nor bacterial proliferation was observed.

Cytokines IL-10 and IL-12 levels were measured by standard Enzyme-Linked Immunosorbent Assay (ELISA) using commercial kits (like Quantikine Kits, R&D Systems Minneapolis, MN), as instructed by the manufacturer, as well known at the skilled person in the art.

Briefly, standards and samples (supernatants from the above co-cultured) were added into the plates and incubated for 2h at room temperature. The specific horseradish peroxidase-conjugated antibody was added to all wells after they were washed 4 times, and the plates were incubated for 1 hour at room temperature. The plates were then washed and incubated for 30 minutes with 3-3',5,5'-tetramethylbenzidine substrate reagent solution. The reaction was stopped by the addition of 1.8 M H₂SO₄. The absorbency of all ELISAs was read at 450 nm with a microtiter plate reader. Standard curves for the cytokines were constructed.

The minimum detectable dose of IL-10 and Il-12 was typically less than 3,9 pg/ml and 5,0 pg/ml, respectively.

Statistical analyses were performed with the Wilcoxon Mann-Whitney test to reveal significant differences between cytokine production in response to different strains of bacteria. Differences were considered to be significant at P<0.05.

### Evaluation of IL-10 and IL-12 production

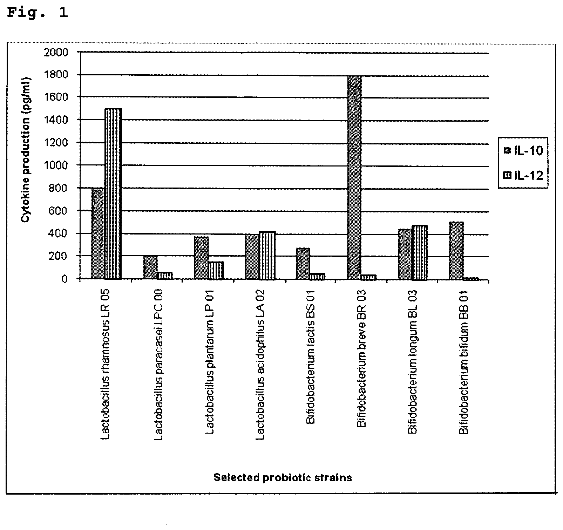

The in vitro immune-stimulation by 5 live bacterial strains of PBMCs collected from healthy donors, revealed distinct capability of the strains to induce IL-10 and IL-12, so that IL-10 and IL-12 levels displayed a strain-specific pattern, as shown in Fig. 1.

The Fig. 1 shows that strain-specific patterns of IL-10 and IL-12 release for different probiotic strains. One experiment representative of 5.

Variations of IL-10 concentrations were substantial with values ranging between 200 and 1700 pg/mL depending on the bacterial strain. For the IL-12 production, we also observed significant variations between strains, covering a range of cytokine levels of 10 to 1200 pg/mL.

*Bifidobacterium breve* BR 03 is able to module the immune responses by inducing the production of IL-10 by in vitro cultured mononuclear cells. *Bifidobacterium breve* BR 03 strongly induced IL-10 production (1688 pg/ml). On the contrary, it has a low capability to stimulate the production of the pro-inflammatory IL-12 (22pg/ml).

The capacity of the probiotic strain *B. breve* BR 03 to boost the production of IL-10 differed considerably between other strains studied, among which can be considered the most potent inducers, see Fig. 1.

In addition to a high IL-10 induction potential, it is important to minimize the IL-12 induction by the probiotic bacteria, when considering selecting a strain for an anti-inflammatory application. The pro-inflammatory cytokine IL-12, is mainly produced by phagocytic and antigen-presenting cells (APCs) as a quick reaction against bacteria, intracellular parasites or other infectious agents. In addition to an important role in the first line of defence against infection, IL-12 will limit or inhibit differentiation of Th2 T cells, itself acting as an immunoregulatory molecule in the Th1 response. IL-12 will induce IFN-γ and directly or indirectly activate natural killer cells, thus enhance further release of pro-inflammatory cytokines which promote an antigen-specific immune response.

This IL-12 production enhancing feedback mechanism, mediated by IFN-γ, is potentially leading to uncontrolled cytokine production. Fortunately, IL-10, as a regulatory cytokine, is a potent inhibitor of IL-12 production by these phagocytic cells and may suppress the emergence of an unbalanced Th1 response, such as the one seen in the gastrointestinal tract of IBD patients in a acute phase of inflammation; hence the importance in selecting probiotic strains with a favorable IL-10/IL-12 ratio.

### Evaluation of IL-10/ IL -12 ratio

It is possible to use the IL-10/IL-12 ratio to distinguish between strains exhibiting a"pro-" *versus* "anti-inflammatory" profile (low *versus* high IL-10/IL-12 ratio, respectively). This approach was found to be useful to identify strains with marked opposite profiles and can be used as a standardized *in vitro* test, allowing preliminary classification of candidate probiotic strains according to their immune modulation capacity that would be predictive of their *in vivo* effect.

The importance of the ratio between these two cytokines was also recently demonstrated by Peran et al.. In the study, administration of a specific strain of *Lactobacillus salivarius* ssp. *salivarius* facilitates the recovery of the inflamed tissue in the TNBS model of rat colitis. This beneficial effect was partly associated to the ability of the strain to modify the cytokine profile in macrophages, reducing the amount of inflammatory cytokine IL-12, while increasing the amount of the anti-inflammatory cytokine IL-10.

The use of PBMC from a diversity of healthy human donors to screen the immunomodulatory activity of candidate probiotic strains by direct stimulation appears to be a good predictive indicator of in vivo anti-inflammatory strains.

Despite the fact that this assay does not clarify the physiological mechanism(s) involved, it seems to mimic how the immune system may sense the bacterial strain and consequently polarise the immune response. Strains leading to a high IL-10/IL-12 ratio would more easily slow down an early Th1 response.

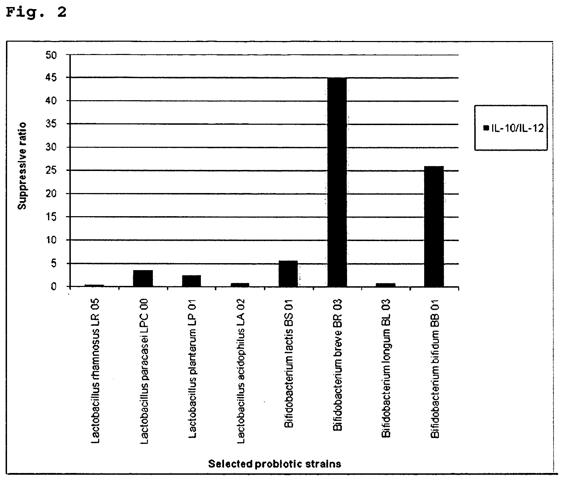

In this context, assessing effects of 5 different probiotic bacteria, we found that *Bifidobacterium breve* BR 03 is the most potent "anti-inflammatory" strain eliciting the best IL-10/IL-12 ratio, as illustrated in Figure 2.

The figure 2 shows that strain-specific IL-10/IL-12 ratio for different probiotic strains. One experiment representative of 5.

Taking into account the above, all the bacteria strains identified in the present invention show:
- a strong capability to induce the anti-inflammatory IL-10 production,
- low capability to stimulate the production of the pro-inflammatory IL-12,
- potent "anti-inflammatory" activity eliciting a high IL-10/IL-12 ratio,
- high persistence in the gastro-intestinal tract due to their resistance to gastric juice, bile salts, pancreatic secretion and to adhesion to gut wall, and
- safe to use having none acquired antibiotic resistances.

## Claims

1. A composition for use in a method of treating Crohn's disease and ulcerative colitis or for use in a method of treating diarrhea and constipation,
wherein the composition is administered orally,
wherein the composition comprises live bacteria, and
wherein the live bacteria belong to a strain selected from the group consisting of *Lactobacillus paracasei* LMG P-21380, *Lactobacillus plantarum* LMG P-21021, *Bifidobacterium lactis* LMG P-21384, and *Bifidobacterium breve* DSM 16604, as inducer of Interleukin-10.

2. The composition for use according to claim 1, wherein said bacterium exhibits a IL-10/IL-12 ratio comprised from bigger than 1 and less than 150, preferably comprised from 10 and 100, more preferably comprised from 30 and 60.

3. The composition for use according to claim 1 or 2, wherein the bacterium strain is *Bifidobacterium breve* DSM 16604 and exhibiting an IL-10/Il-12 ratio which is comprised from 50 and 100, preferably from 70 and 80E.

4. The composition for use according to claim 1 or 2, wherein the live bacteria are the bacteria strain *Bifidobacterium breve* DSM 16604 in combination with at least one bacteria strain selected from the group consisting of *Lactobacillus paracasei* LMG P-21380, *Lactobacillus plantarum* LMG P-21021 and *Bifidobacterium lactis* LMG P-21384.

## Patentansprüche

1. Zusammensetzung zur Verwendung in einem Verfahren zur Behandlung von Morbus Crohn und Colitis ulcerosa oder zur Verwendung in einem Verfahren zur Behandlung von Durchfall und Verstopfung,
wobei die Zusammensetzung oral verabreicht wird,
wobei die Zusammensetzung lebende Bakterien umfasst, und
wobei die lebenden Bakterien als Induktoren von Interleukin-10 zu einem Stamm ausgewählt aus der Gruppe bestehend aus *Lactobacillus paracasei* LMG P-21380, *Lactobacillus plantarum* LMG P-21021, *Bifidobacterium lactis* LMG P-21384 und *Bifidobacterium breve* DSM 16604 gehören.

2. Zusammensetzung zur Verwendung nach Anspruch 1, wobei das Bakterium ein IL-10-/IL-12-Verhältnis von mehr als 1 bis weniger als 150, vorzugsweise von 10 bis 100, noch bevorzugter von 30 bis 60, aufweist.

3. Zusammensetzung zur Verwendung nach Anspruch 1 oder 2, wobei der Bakterienstamm zum Stamm *Bifidobacterium breve* DSM 16604 gehört und ein IL-10-/IL-12-Verhältnis von 50 bis 100, vorzugsweise von 70 bis 80 E, aufweist.

4. Zusammensetzung zur Verwendung nach Anspruch 1 oder 2, wobei die lebenden Bakterien der Bakterienstamm *Bifidobacterium breve* DSM 16604 sind, in Kombination mit wenigstens einem Bakterienstamm, der aus der Gruppe bestehend aus *Lactobacillus paracasei* LMG P-21380, *Lactobacillus plantarum* LMG P-21021 und *Bifidobacterium lactis* LMG P-21384 ausgewählt wird.

## Revendications

1. Composition pour une utilisation dans une méthode de traitement de la maladie de Crohn et de la recto-colite hémorragique ou pour une utilisation dans une méthode de traitement d'une diarrhée et d'une constipation,
laquelle composition est administrée par voie orale,
laquelle composition comprend des bactéries vivantes, et
dans laquelle les bactéries vivantes appartiennent à une souche choisie dans le groupe constitué par *Lactobacillus paracasei* LMG P-21380, *Lactobacillus plantarum* LMG P-21021, *Bifidobacterium lactis* LMG P-21384, et *Bifidobacterium breve* DSM 16604, en tant qu'inducteur d'interleukine-10.

2. Composition pour une utilisation selon la revendication 1, dans laquelle ladite bactérie présente un rapport IL-10/IL-12 compris entre plus de 1 et moins de 150, de préférence compris entre 10 et 100, mieux encore compris entre 30 et 60.

3. Composition pour une utilisation selon la revendication 1 ou 2, dans laquelle la souche bactérienne est *Bifidobacterium breve* DSM 16604 et présente un rapport IL-10/IL-12 qui est compris entre 50 et 100, de préférence entre 70 et 80E.

4. Composition pour une utilisation selon la revendication 1 ou 2, dans laquelle les bactéries vivantes sont de la souche bactérienne *Bifidobacterium breve* DSM 16604 en combinaison avec au moins une souche bactérienne choisie dans le groupe constitué par *Lactobacillus paracasei* LMG P-21380, *Lactobacillus plantarum* LMG P-21021 et *Bifidobacterium lactis* LMG P-21384.
